(19) Europäisches Patentamt
European Patent Office
Office européen des brevets

(11) Veröffentlichungsnummer: **0 067 360**
**B1**

(12) # EUROPÄISCHE PATENTSCHRIFT

(45) Veröffentlichungstag der Patentschrift: **22.05.85**

(51) Int. Cl.[4]: **C 07 D 207/323** // B01J23/00

(21) Anmeldenummer: **82104788.3**

(22) Anmeldetag: **01.06.82**

(54) **Verfahren zur Herstellung von Pyrrolen.**

(30) Priorität: **12.06.81 DE 3123302**

(43) Veröffentlichungstag der Anmeldung: **22.12.82 Patentblatt 82/51**

(45) Bekanntmachung des Hinweises auf die Patenterteilung: **22.05.85 Patentblatt 85/21**

(84) Benannte Vertragsstaaten: **AT BE CH DE FR GB IT LI NL**

(56) Entgegenhaltungen:
**GB - A - 515 865**
**GB - A - 832 855**
**GB - A - 1 393 086**
**US - A - 3 008 965**
**US - A - 3 522 269**

(73) Patentinhaber: **BASF Aktiengesellschaft, Carl-Bosch-Strasse 38, D-6700 Ludwigshafen (DE)**

(72) Erfinder: **Goetz, Norbert, Dr., Schoefferstrasse 25, D-6520 Worms (DE)**
Erfinder: **Hupfer, Leopold, Dr., Waltershoehe 3, D-6701 Friedelsheim (DE)**
Erfinder: **Franzischka, Wolfgang, Dr., Franz-Lind-Strasse 1, D-6713 Freinsheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von Pyrrolen durch Umsetzung von Pyrrolidinen bei 160 bis 400 °C in Gegenwart von Palladiumträgerkatalysatoren, die basische Verbindungen und/oder Elemente der Gruppe Ib, IIb, VIIb, Cobalt und/oder Nickel enthalten.

Es ist bekannt, dass Heterocyclen, die hydrierte Stufen von heteroaromatischen Verbindungen darstellen, durch Dehydrierung an Palladium- oder Platin-Trägerkatalysatoren, z.B. bei 300 bis 350 °C in die entsprechende Heteroaromaten überführt werden können [Houben-Weyl, Methoden der Organischen Chemie, Band 4/2, Seiten 338-341, N.D. Zelinsky, J.K. Jurjew, Chem. Ber., Band 64 (1931), Seiten 101-103]. Besonders hervorgehoben werden Palladiumkatalysatoren auf Kieselgelträgern für die Dehydrierung, z.B. bei 350-500 °C, von gesättigten heterocyclischen Verbindungen zu Heteroaromaten (Britische Patentschrift 1 393 086), speziell von Pyrrolidinen zu Pyrrolen (US-Patentschrift 3 522 269). Auffallend ist, dass bei Verwendung von Silikagel als Trägermaterial relativ hohe Reaktionstemperaturen (350 - 500 °C) angewendet werden müssen, um befriedigende Umsätze zu erreichen. Die Umsetzung von Pyrrolidin und N-substituierten Homologen des Pyrrolidins zu den entsprechenden Pyrrolen an Palladiumkatalysatoren auf Aluminiumoxidträgern wird in der US-Patentschrift 3 008 965 beschrieben. Nach dieser Arbeitsweise werden im Temperaturbereich von 175 - 350 °C Umsätze zwischen 45 und 65% erreicht, die nach 12 bis 15 Stunden Betriebszeit zu sinken beginnen, wobei der Abfall des Umsatzes durch stufenweise Steigerung der Reaktionstemperatur auf 350 °C abgefangen wird. Palladiumkatalysatoren auf gemischten $SiO_2$-$Al_2O_3$-Trägern sind nach den Angaben in der vorher zitierten US-Patentschrift weniger aktiv als Palladiumkatalysatoren auf reinen $Al_2O_3$-Trägern.

Es wurde nun gefunden, dass man Pyrrole der Formel

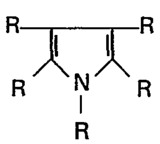

I

worin die einzelnen Reste R gleich oder verschieden sind und jeweils ein Wasserstoffatom oder einen aliphatischen Rest bedeuten, durch Umsetzung von Pyrrolidinen der Formel

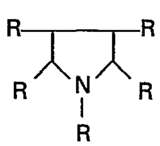

II

worin R die vorgenannte Bedeutung besitzt, bei Temperaturen von 160 bis 400 °C vorteilhaft erhält, wenn man die Umsetzung in Gegenwart von Palladiumträgerkatalysatoren, die basische Verbindungen der Alkali-, Erdalkalimetalle und/oder Metalle der seltenen Erden und/oder Elemente der Gruppe Ib, IIb, VIIb, Cobalt und/oder Nickel enthalten, durchführt.

Weiterhin wurde gefunden, dass man das Verfahren vorteilhaft durchführt, wenn man in Anwesenheit von tertiären Aminen umsetzt.

Die Umsetzung kann unter Verwendung von N-Methylpyrrolidin durch die folgenden Formeln wiedergegeben werden:

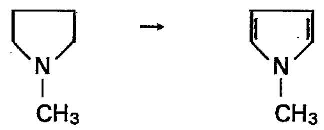

+ $2H_2$ .

Die Vorteile des erfindungsgemässen Verfahrens bestehen darin, dass Katalysatoren zur Anwendung kommen, die es erlauben, bei Temperaturen unterhalb von 300 °C fast vollständige Umsätze der eingesetzten Pyrrolidine zu den gesuchten Pyrrolen zu erreichen, wobei die Pyrrole in ausgezeichneten Ausbeuten und guter Reinheit anfallen. Vorteilhaft ist insbesondere, dass die beanspruchten Katalysatoren eine sehr lange Lebenszeit haben, so dass selbst nach einer Dauerbeanspruchung von über 1200 Betriebsstunden kein Aktivitätsabfall zu verzeichnen ist (siehe Beispiel 1). Sollten trotz dieser weitgehenden Unempfindlichkeit der erfindungsgemässen Katalysatoren Beeinträchtigungen der Aktivität vorkommen, die beispielsweise durch Zufuhr verunreinigter Ausgangsprodukte verursacht sein können, lässt sich durch Abbrennen bei 300 °C und anschliessende Aktivierung mit Wasserstoff bei 300 °C eine vollständige Regenerierung herbeiführen. Bei Arbeiten mit Temperaturen oberhalb von 300 °C erwiesen sich die verwendeten Katalysatoren als sehr stabil und langlebig, obwohl keine Notwendigkeit besteht, in diesem hohen Temperaturbereich zu arbeiten, da unterhalb von 300 °C quantitative Umsätze und sehr gute Ausbeuten zu erzielen sind. Verzichtet man auf hohe Umsatzraten und führt den nicht umgesetzten Ausgangsstoff wieder in die Reaktionszone zurück, sind Reaktionstemperaturen um 200 °C und darunter ausreichend.

Die Bildung von Nebenprodukten, wie beispielsweise von instabilen Pyrrolinen, die durch Folgereaktionen (z.B. Polymerisationen) für höhermolekulare Ablagerungen auf der Katalysatoroberfläche verantwortlich sein können, Crack- oder Sintervorgänge, die ebenfalls zu Veränderungen an der Oberfläche von Katalysatoren führen können, werden nicht beobachtet. Alle diese vorteilhaften Ergebnisse des erfindungsgemässen Verfahrens sind im Hinblick auf den Stand der Technik überraschend.

Bevorzugte Ausgangsstoffe II und dementsprechend bevorzugte Endstoffe I sind solche, in deren Formeln die einzelnen Reste R gleich oder verschieden sein können und jeweils ein Wasserstoffatom oder einen Alkylrest mit 1 bis 10, vorteilhaft 1 bis 6 Kohlenstoffatomen bedeuten. Die vorgenannten Reste können noch durch unter den Reaktionsbedingungen inerte Gruppen, z.B. Alkyl- oder Alkoxygruppen mit jeweils 1 bis 4 Kohlenstoffatomen, substituiert sein.

So kommen beispielsweise folgende Ausgangsstoffe II in Betracht: Pyrrolidin; in 1-, 2-, 3-, 4-, 5-Stellung durch die Methyl-, Ethyl-, Propyl-, Isopropyl-, Butyl-, Isobutyl-, sek.-Butyl-, tert.-Butyl-, Pentyl-, Hexylgruppe substituierte Pyrrolidine; in 1,2-, 1,3-, 2,4-, 2,5-, 2,3-Stellung durch vorgenannte Substituenten substituierte Pyrrolidine; in 1,2,3-, 1,3,4-, 1,2,5-, 1,2,4-, 2,3,4-, 3,4,5-Stellung durch vorgenannte Substituenten substituierte Pyrrolidine; homologe in 1,2,3,4-, 1,2,3,5-, 2,3,4,5-Stellung substituierte Pyrrolidine; in allen Stellungen substituierte Pyrrolidine; bevorzugt sind Pyrrolidin, N-Methylpyrrolidin, 2-Methylpyrrolidin, 3-Methylpyrrolidin, N-Ethylpyrrolidin, N-Hexylpyrrolidin, N-Decylpyrrolidin, 2,5-Dimethylpyrrolidin, 2,4-Dimethylpyrrolidin, 2,3,4-Trimethylpyrrolidin, 2,3,4,5-Tetramethylpyrrolidin, 2,5-Diethylpyrrolidin, 2,5-Di--n-butylpyrrolidin.

Die Umsetzung wird bei einer Temperatur von 160 bis 400°C, vorzugsweise 160 bis 300°C, insbesondere 200 bis 300°C, drucklos oder unter Druck, diskontinuierlich oder bevorzugt kontinuierlich durchgeführt. Die Pyrrolidine II können lösungsmittelfrei oder in einem Lösungsmittel gelöst, das unter den Reaktionsbedingungen indifferent ist, der Reaktionszone zugeführt werden. Als Lösungsmittel kommen beispielsweise in Frage: aromatische Kohlenwasserstoffe, z.B. Toluol, Ethylbenzol, o-, m-, p-Xylol, Isopropylbenzol; Alkanole und Cycloalkanole wie Ethanol, Methanol, n-Butanol, Isobutanol, tert.-Butanol, n-Propanol, Isopropanol, Amylalkohol, Cyclohexanol, 2-Methyl-4-pentanol, Ethylenglykolmonoethylether, 2-Ethylhexanol, Methylglykol, n-Hexanol, Isohexylalkohol, Isoheptylalkohol, n-Heptanol, Ethylbutanol, Nonylalkohol, Dodecylalkohol, Methylcyclohexanol, insbesondere solche mit 1 bis 4 Kohlenstoffatomen; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan; bevorzugt sind Toluol, Xylole, Diethylbenzol, Ethanol, n-Butanol, Methylglykol, Ethylglykol, 1,2-Dimethoxyethan; und entsprechende Gemische. Zweckmässig verwendet man das Lösungsmittel in einer Menge von 80 bis 10 000 Gewichtsprozent, vorzugsweise von 100 bis 600 Gewichtsprozent, bezogen auf Ausgangsstoff II.

Als basische Verbindungen werden im allgemeinen Verbindungen der Alkali-, Erdalkalimetalle und/oder Metalle der seltenen Erden verwendet. Es können aber auch tertiäre Amine und basische Zinkverbindung verwendet werden. Flüchtige Basen werden zweckmässig zusammen mit dem Ausgangsstoff der Reaktionszone zugeführt. So können beispielsweise tert.-Amine nach dem erfindungsgemässen Verfahren auch noch gleichzeitig als Lösungsmittel verwendet werden. Es kommen z.B. als basische Verbindungen in Frage:
Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumoxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Natriumpropionat, Natriumbutyrat, Natriumisobutyrat, Kaliumformiat, Kaliumacetat, Kaliumpropionat, Kaliumbutyrat, Kaliumisobutyrat, Natriummethylat, Natriumethylat, Natriumpropylat, Natriumisopropylat, Natriumbutylat, Natriumisobutylat, Natrium-sek.-butylat, Natrium-tert.-butylat, Natriumethylenglykolat, Natriumpropylen-(1,2)-glykolat, Natriumpropylen-(1,3)-glykolat, Natriumdiethylenglykolat, Natriumtriethylenglykolat, Natriumdipropylen-(1,2)-glykolat, Kaliumethylat, Kaliumethylat, Kalium-n-propylat, Kaliumisopropylat, Kalium-n-butylat, Kaliumisobutylat, Kalium-sek.-butylat, Kalium-tert.-butylat, Kaliumethylenglykolat, Kaliumpropylen-(1,2)-glykolat, Kaliumpropylen--(1,3)-glykolat, Kaliumdiethylenglykolat, Kaliumtriethylenglykolat, Kaliumdipropylen-(1,2)-glykolat; Trimethylamin, Triethylamin, Tripropylamin, Triisopropylamin, Tributylamin, Triisobutylamin, tri-sek.-butylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N-Methylpiperidin, N-Ethylpiperidin, N-Methylmorpholin, N-Ethylmorpholin, N-Methylhexamethylenimin, N-Ethylhexamethylenimin. Bevorzugt sind die Oxide, Hydroxide oder Carbonate der Alkalimetalle, vorzugsweise des Lithiums, der Erdalkalimetalle, vorzugsweise des Magnesiums und Calciums, der Seltenerdmetalle, vorzugsweise des Cers, Praseodyms und Neodyms. Die Umsetzung wird zweckmässig in Gegenwart einer basischen Verbindung in einer Menge von 0,001 bis 10 000, vorzugsweise von 0,01 bis 1000 Gew.-% basischer Verbindung, bezogen auf die Gewichtsmenge von Ausgangsstoff II, durchgeführt.

Als Zusatzelemente verwendet man solche der Gruppe Ib, z.B. Kupfer, Silber, IIb Zink oder Cadmium, VIIb, z.B. Mangan, Cobalt und/oder Nickel; bevorzugt sind Nickel, Cobalt, Mangan, Zink und Silber. Zweckmässig ist das Gewichtsverhältnis von 0,001 bis 1000 : 1, bevorzugt 0,01 bis 100 : 1 Zusatzelement, bezogen auf den Ausgangsstoff II.

Der Palladiumgehalt des Katalysators, bezogen auf das Trägermaterial, liegt in der Regel bei 0,05 bis 15 Gewichtsprozent. Das Gewichtsverhältnis der Zusätze zu Palladium kann zweckmässig von 0,02 bis 10 000 : 1, bevorzugt 0,02 bis 100 : 1 betragen. Man verwendet den Katalysator zweckmässig in Form von Strängen mit einem Durchmesser von 3 mm und einer Länge von 10 mm oder als Pulver (Wirbelkorn). Zweckmässig wählt man Gewichtsverhältnisse von 0,001 bis 1000 : 1, vorzugsweise 0,01 bis 100 : 1 Palladium, bezogen auf den Ausgangsstoff II.

Die Reaktion kann wie folgt durchgeführt werden: Ein Gemisch von Ausgangsstoff II und Palladiumträgerkatalysator, gegebenenfalls zusammen mit Lösungsmittel, wird während 1 bis 10 Stunden bei der Reaktionstemperatur gehalten. Aus dem Rektionsgemisch wird der Endstoff I in üblicher Weise, z.B. durch Filtration und fraktionierte Destillation, abbetrennt. Das Verfahren wird in einer bevorzugten Ausführungsform kontinuierlich in der Gasphase durchgeführt, wobei der Katalysator in einem Festbett oder als Wirbelbett angeordnet sein kann. Die Ausgangsprodukte können zusammen mit einem

Trägergas, wie beispielsweise Stickstoff oder vorzugsweise Wasserstoff, über den Katalysator geleitet werden, wobei das Verhältnis von Pyrrolidin zu Trägergas zweckmässig in Molverhältnissen von 1 : 1 bis 1 : 20 variiert werden kann.

Das nach dem Verfahren der Erfindung verwendete Katalysatorsystem enthält einerseits Palladium und andererseits aktive Zusätze, wobei die Zusätze allein oder im Gemisch miteinander neben dem Palladium vorliegen können. Die aktiven Bestandteile des Katalysators sind auf einem Träger aufgebracht, der aus Aluminiumoxid, Kieselsäure, Aluminiumsilikat, Magnesiumsilikat, Aktivkohle oder Spinellen des Aluminiums, Chroms oder Eisens bestehen kann, wobei die Zusätze, die dem Katalysator seine Eigenschaften verleihen, sich an der Oberfläche des Katalysators befinden können oder zusammen mit einem indifferenten Trägermaterial in Form einer Mischung den Träger darstellen können oder mit dem ursprünglichen Träger durch nachträgliches Tempern ein gemeinsames Kristallgitter bilden können; beispielsweise ergibt sich bei Aluminiumoxidträgern die Bildung von typischen Spinellgittern mit Oxiden bestimmter Metalle, z.B. Li, Mg, Zn, Mn, Co. Die Aktivität und die Lebensdauer der Katalysatoren werden sehr wesentlich durch die im Träger enthaltenen Zusätze beeinflusst. Vorzugsweise wird Aluminiumoxid als Träger verwendet. Die Zusätze können gemeinsam mit dem Palladium durch Tränken des Trägermaterials mit Lösungen von z.B. Nitraten, Chloriden, Carbonaten, Formiaten oder Oxalaten aufgebracht werden. Durch anschliessende thermische Behandlung, die zweckmässig zwischen 400 und 600°C durchgeführt wird, erfolgt die Überführung in die Oxidform. Soll bei Aluminiumoxidträgern mit geeigneten Metallen (Mg, Zn, Co, Mn, Li) Spinellbildung erzielt werden, muss zweckmässig nach der Tränkung auf eine Temperatur von 900 bis 1300°C erhitzt werden und anschliessend das Palladium aufgebracht werden. Manche Trägerzusätze, wie beispielsweise Calciumoxid oder Magnesiumoxid, lassen sich mit einem Träger wie Aluminiumoxid mischen und bilden dann nach gemeinsamen Tempern bei 400 bis 600°C einen neuen Trger, auf dem das Palladium niedergeschlagen werden kann.

Vorteilhafte Herstellmöglichkeiten für den Katalysator sind:

a) Auf strang- oder pulverförmiges $\gamma$-Aluminiumoxid wird Palladium zusammen mit Zusatz (Mangan-, Zink-, Silber-, Seltenerdmetallverbindungen (basische Verbindungen) in der gewünschten Menge durch Tränken mit beispielsweise deren Nitratlösungen und anschliessendes Eindampfen bis zur Trockne aufgebracht. Danach wird 6 Stunden bei 550°C getempert und im Wasserstoffstrom bei 200°C reduziert.

b) Auf strang- oder pulverförmiges $\gamma$-Aluminiumoxid wird zunächst die gewünschte Menge des Zusatzes (Mangan-, Zink-, Cobalt-, Magnesium-, Lithiumverbindungen) durch Tränken mit entsprechenden wässrigen Nitrat- oder Formiatlösungen aufgebracht und bei 150°C getrocknet. Der derart vorbehandelte Träger wird nun entweder 6 Stunden lang auf 550°C erhitzt oder, wenn Spinellbildung erreicht werden soll, 6 Stunden lang bei 1050°C geglüht. Nun wird der Träger mit wässriger Palladiumnitratlösung getränkt und durch 7stündiges Erhitzen bei 200°C im Wasserstoffstrom reduziert. Wurde zum Tränken Palladium-(II)-chloridlösung genommen, wird zweckmässig mit alkalischer Formalinlösung oder 5prozentiger Hydrazinlösung reduziert.

c) $\gamma$-Aluminiumoxid und ein basisches Oxid (MgO, CaO) werden in dem gewünschten Mengenverhältnis intensiv miteinander vermischt (beispielsweise in einem Kneter). Diese Mischung wird 6 Stunden lang auf 450°C erhitzt, anschliessend mit Palladiumnitratlösung getränkt und 7 Stunden lang bei 300°C im Wasserstoffstrom reduziert.

Die nach dem Verfahren der Erfindung hergestellten Pyrrole werden z.B. als Korrosionsinhibitoren (US-PS 3 008 965), zur Herstellung von Schädlingsbekämpfungsmitteln, Farbstoffen (Kirk-Othmer Encycl. Chem. Technol., 2nd Ed. 1968, Band 16, Seiten 841-858; Ullmanns Encycl. d. techn. Chem. (1963), Band 14, Seiten 505-510) oder Arzneimitteln (FR-PS 1 574 570) verwendet. Bezüglich der Verwendung wird auf vorgenannte Veröffentlichungen verwiesen.

*Beispiel 1*

In einem Rohrreaktor mit einem Fassungsvermögen von 1,2 Liter wurde ein Katalysator in Form von Strängen (3 mm Durchmesser , 10 mm Länge), der 0,5 Gewichtsprozent Palladium auf einer Mischung aus 19,4 Gewichtsprozent Magnesiumoxid und 80,6 Gewichtsprozent Aluminiumoxid enthält, eingefüllt und auf 250°C erhitzt. Über dieses Katalysatorbett wurden stündlich 100 Gramm N-Methylpyrrolidin und 50 Liter Wasserstoff pro Stunde im Gleichstrom hindurchgeleitet. Das Reaktionsprodukt wurde, sobald es den Reaktor verliess, abgekühlt. Es bestand nach gaschromatographischer Analyse aus 98,5 Gewichtsprozent N-Methylpyrrol und 1,5 Gewichtsprozent N-Methylpyrrolidin. Nach einer destillativen Aufarbeitung des Reaktionsproduktes erhielt man aus 100 Gramm Ausgangsstoff 94,5 Gramm N-Methylpyrrol (Kp 1013 mbar = 115°C) in einer Ausbeute von 97% der Theorie. Der Katalysator zeigte nach 1200 Stunden Betriebszeit noch keinen Aktivitätsverlust.

*Beispiel 2*

Man verfuhr entsprechend Beispiel 1, verwendete aber einen Katalysator, der 0,5 Gewichtsprozent Palladium auf 19,4 Gewichtsprozent Calciumoxid und 80,6 Gewichtsprozent Aluminiumoxid enthielt. Man erhält aus 100 Gramm Ausgangsstoff 96 Gramm Endstoff (99% der Theorie) vom Kp 115°C/1013 mbar.

*Beispiel 3*

Man verfuhr entsprechend Beispiel 1, verwendete aber einen Katalysator, der 1,0 Gewichtsprozent Palladium und 0,5 Gewichtsprozent Praseodymoxid auf Aluminiumoxid enthielt und arbeitete bei einer Reaktionstemperatur von 230°C. Als Ausgangsstoff wurde Pyrrolidin genommen, das unter den angegebenen Reaktionsbedingungen zu 93 Gewichtsprozent zum Pyrrol (Kp 1013 mbar = 130°C) (93%

der Theorie) umgesetzt wurde, der Rest bestand aus unumgesetztem Ausgangsstoff (Selektivität: praktisch 100%).

*Beispiel 4*

Man verfuhr entsprechend Beispiel 1, verwendete aber einen Katalysator, der 1,0 Gewichtsprozent Palladium auf Lithium-Aluminiumspinell enthielt und hielt eine Reaktionstemperatur von 275°C ein. N-Hexylpyrrolidin wurde unter diesen Bedingungen zu 98,3 Gewichtsprozent zum N-Hexylpyrrol (98,3% der theorie) (Kp 1013 mbar = 213°C) umgesetzt.

*Beispiel 5*

Man verfuhr entsprechend Beispiel 1, verwendete aber einen Katalysator, der 1,0 Gewichtsprozent Palladium auf Cobalt-Aluminiumspinell enthielt und arbeitete bei einer Reaktionstemperatur von 275°C. 2-Methylpyrrolidin wurde unter diesen bedingungen zu 97,7 Gewichtsprozent zum 2-Methylpyrrol (97,7% der Theorie) (Kp 1013 mbar = 148°C) umgesetzt.

*Beispiel 6*

Man verfuhr entsprechend Beispiel 1, verwendete aber einen Katalysator, der 1,0 Gewichtsprozent Palladium auf Zink-Aluminiumspinell enthielt und arbeitete bei einer Reaktionstemperatur von 275°C. 3-Methylpirrolidin wurde unter diesen Bedingungen zu 95,8 Gewichtsprozent zum 3-Methylpyrrol (95,8% der Theorie) (Kp 986 mbar = 143°C) umgesetzt.

*Beispiel 7*

Man verfuhr entsprechend Beispiel 1, verwendete aber einen Katalysator, der 1,0 Gewichtsprozent Palladium auf Magnesium-Aluminiumspinell enthielt und arbeitete bei einer Reaktionstemperatur von 275°C. 2,4-Dimethylpyrrolidin wurde unter diesen Reaktionsbedingungen zu 98,7 Gewichtsprozent zum 2,4-Dimethylpyrrol (98,7% der Theorie) (Kp 1013 mbar = 165°C) umgesetzt.

*Beispiel 8*

In einen Wirbelbettreaktor mit einem Fassungsvermögen von 1,3 Liter wurde 1 Liter Katalysator eingefüllt. Der Katalysator hatte die Zusammensetzung 0,5 Gewichtsprozent Palladium, 1,0 Gewichtsprozent Mangan, 5,0 Gewichtsprozent Cer-(IV)-oxid auf Aluminiumoxid und besass eine Korngrösse von 0,2 bis 0,6 mm. Die Temperatur des Reaktors wurde auf 230°C eingestellt und eine entsprechend vorerhitzte Mischung aus 100 Liter Wasserstoff und 100 Liter Stickstoff pro Stunde eingeleitet. Durch die so erzeugte Katalysator-Wirbelschicht wurden stündlich 100 Gramm gasförmiges N-Methylpyrrolidin geführt. Das durch Abkühlung gewonnene Reaktionsprodukt bestand aus 96,7 Gewichtsprozent N-Methylpyrrol (96,7% der Theorie) und 3,3 Gewichtsprozent N-Methylpyrrolidin.

*Beispiel 9*

Man verfuhr entsprechend Beispiel 8, verwendete aber einen Katalysator, der 0,5 Gewichtsprozent Palladium und 5,0 Gewichtsprozent Silber auf Aluminiumoxid enthielt. N-Methylpyrrolidin wurde unter diesen Bedingungen zu 94,5 Gewichtsprozent in N-Methylpyrrol (94,5% der Theorie) umgewandelt.

*Beispiel 10*

Man verfuhr entsprechend Beispiel 1, setzte aber stündlich als Ausgangsstoff eine 50prozentige Lösung von N-Methylpyrrolidin in N-Methylmorpholin (als basische Verbindung) ein. Aus 100 Gramm N-Methylpyrrolidin wurden nach dieser Verfahrensweise 96 Gramm N-Methylpyrrol (99% der Theorie) gebildet.

**Patentansprüche**

1. Verfahren zur Herstellung von Pyrrolen der Formel

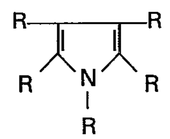

I

worin die einzelnen Reste R gleich oder verschieden sind und jeweils ein Wasserstoffatom oder einen aliphatischen Rest bedeuten, durch Umsetzung von Pyrrolidinen der Formel

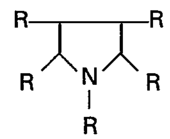

II

worin R die vorgenannte Bedeutung besitzt, bei Temperaturen von 160 bis 400°C, dadurch gekennzeichnet, dass man die Umsetzung in Gegenwart von Palladiumträgerkatalysatoren, die basische Verbindungen der Alkali-, Erdalkalimetalle und/oder Metalle der seltenen Erden und/oder Elemente der Gruppe Ib, IIb, VIIb, Cobalt und/oder Nickel enthalten, durchführt.

2. Verfahren gemäss Anspruch 1, dadurch gekennzeichnet, dass man in Anwesenheit von tertiären Aminen umsetzt.

**Claims**

1. A process for the preparation of a pyrrole of the formula

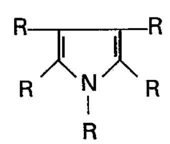

I

where the individual radicals R are identical or different and each is hydrogen or an aliphatic radical, by reacting a pyrrolidine of the formula

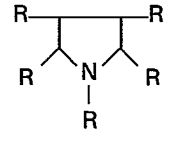

II

where R has the above meaning, at from 160 to 400°C, wherein the reaction is carried out in the presence of a supported palladium catalyst which contains a basic compound of an alkali metal, an alkaline earth metal and/or a rare earth metal and/or an element of group Ib, IIb or VIIb, cobalt and/or nickel.

2. A process as claimed in claim 1, wherein the reaction is carried out in the presence of a tertiary amine.

## Revendications

1. Procédé pour la préparation de pyrroles de formule

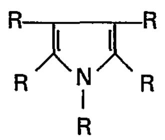

I

dans laquelle les différents radicaux R peuvent être semblables ou dissemblables et représentent chacun un atome d'hydrogène ou un radical aliphatique, par transformation de pyrrolidines de formule

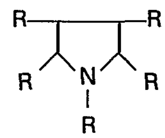

II

dans laquelle R a la signification donnée ci-dessus, à des températures de 160 à 400°C, caractérisé en ce qu'on mène la réaction en présence de catalyseurs au palladium fixés sur support, contenant des composés basiques des métaux alcalins, alcalino-terreux et/ou de métaux des terres rares et/ou des éléments des groupes Ib, IIb, VIIb, du cobalt et/ou du nickel.

2. Procédé selon la revendication 1, caractérisé en ce qu'on mène la réaction en présence d'amines tertiaires.